# NEW EUROPEAN PATENT SPECIFICATION

(11) **EP 3 989 888 B2**
(45) Date of publication and mention of the opposition decision: **29.07.2026**
(45) Mention of the grant of the patent: 25.10.2023
(21) Application number: 20736570.1
(22) Date of filing: 25.06.2020
(51) Int. Cl.: A61F 5/44, A61F 5/445

(54) **AN OSTOMY APPLIANCE**
EINE STOMAVORRICHTUNG
UN APPAREILLAGE DE STOMIE

(30) Priority: 26.06.2019 DK PA201970405
(43) Date of publication of application: 04.05.2022
(73) Proprietor: Coloplast A/S, 3050 Humlebaek (DK)
(72) Inventor: MOELLER, Niels Peder, 2800 Kongens Lyngby (DK); HICKMOTT, Richard Morgan, 3000 Helsingoer (DK); NIELSEN, Torben Holst, 2640 Hedehusene (DK)
(86) International application number: PCT/DK2020/050194
(87) International publication number: WO 2020/259784

(56) References cited:
- EP-A1- 1 991 187
- WO-A1-2015/014774
- US-A1- 2019 142 623
- US-B1- 6 171 289
- US-B2- 7 737 321

## Description

The disclosure concerns a base plate for an ostomy appliance. The base plate forms a proximal surface configured to be attached to skin surface of the body and defines a stoma-receiving opening from the proximal surface, through the base plate to allow communication into a stomal output collecting bag which can be attached to, or which may form part of the base plate. The ostomy appliance further comprises an electrode structure with at least two electrodes which can be used e.g. for detection of humidity.

### BACKGROUND

Stomal output often contains body fluids and visceral contents that are aggressive to both the skin of a user and to ostomy devices. In particular these may have a detrimental effect on the efficiency and integrity of the adhesive materials that are applied to attach the ostomy device to the user's skin surface. Some ostomists may wear their device for prolonged periods of time. For users in general - and for these ostomists in particular - safe, reliable and efficient ostomy devices are highly desirable.

Attempts have been made to reduce or eliminate the unpleasant consequences of leakage, and electric leak detection sensors are developed for notifying the user when humidity at the interface between the skin surface and the ostomy device exceeds a limit.

Unfortunately, such sensors may provide false signals and therefore create unnecessary concern for the ostomist.

EP1991187, which discloses the preamble of claim 1, discloses a sensor assembly for an ostomy appliance.

US 2019/142623 A1 relates to a thermoresponsive skin barrier assembly including a wound treatment assembling having a pump configured to expel a biosealant to a pump output port when subjected to a thermal stimulus, and an adhesive substrate for covering the wound. Ostomists and health care professionals alike would welcome improvements in ostomy devices to better meet requirements related to reliable and fast indication of leakage.

### SUMMARY

To improve the ability to detect humidity, to provide a more reliable signal from the electrode of an ostomy appliance, and to facilitate better ways of manufacturing advanced ostomy appliances, the disclosure, in a first aspect, provides an ostomy appliance wherein the electrode structure extends in a recess structure which defines a volume, *v1,* and where *v1* exceeds the volume, *v2,* of the electrode to thereby define a free space with a volume, *v3,* around the electrode structure.

Due to the free volume, *v3,* the electrode will be less prone to react on humidity caused by perspiration on the skin surface to which the proximal surface is attached. The free space allows accumulation of humidity and facilitates an intermediate storage for leaking liquid before it escapes to the ambience. Accordingly, the free space improves the ability to detect liquids and to distinguish between liquids caused by leakage from liquids caused by perspiration, and the reliability may therefore be improved.

The base plate could be made as a planar element or as a convex element. The base plate could be made from a base composition including one, or some, or all, of the following materials: one or more polyethylene based polymers, one or more ethylene vinyl acetate-based polymers, one or more thermoplastic hydrocolloids, one or more hydrocolloid powders, one or more hydrophilic polyurethane-based polymers, one or more filler materials and/or one or more water swellable/soluble polymers like polyvinylalcohols (PVOH) and copolymers thereof and/or polylactic acids (PLA).

The proximal surface is to be attached to the body and defines the stoma-receiving opening. On an opposite, distal, surface, the base plate may be attachable to or it may be formed in one part with a stomal output collecting bag arranged to be in communication with the stoma-receiving opening.

The electrodes can be formed of many different types of materials, for example metals like silver, gold, aluminium or copper or paste of silver or aluminium; conducting polymers like polyaniline, polypyrrole, ethylenedioxythiophene, poly(p-pyridyl vinylene); or amorphous conducting carbon films, films of conducting carbon fibres or polymer-conducting-carbon-black. Materials may also be alloys and/or semiconductors such as tin oxide (SnO2), zinc oxide (ZnO2), indium tin oxide (ITO) or the like. In one embodiment, the electrodes have structural integrity. They may e.g. be solid, rigid pieces or filament of metal, e.g. with a circular cross-section, e.g. made as wires. The solid and rigid 3-dimensional shape of such wires or filaments enables the application of the electrodes by pressing them into a less rigid adhesive of the proximal surface. The electrodes may be formed in many different forms and shapes. For example, they may be formed as tracks having many different paths, for example any known geometrical shape such as oval, square, triangular or alternatively have an irregular shape. In one preferred embodiment the first and the second electrode are formed as respective first and second circular track and where the inner diameter of the second circular track is larger than the outer diameter of the first circular track. This allows the electrodes to be simple to produce and allows signals representing a leak to be generated irrespective of the position where the leak occurs along the electrodes.

In one embodiment, the electrodes are formed as intertwined wires e.g. of copper, or of a polymer material comprising a conductive material such as copper or carbon fibres.

The electrodes may be coated with an electrically insulating polymer coating. The coating of the wires, and particularly of intertwined wires, may be provided with spaced holes, to allow liquid or humidity etc. to enter a first hole of one of the two electrodes and a first hole of the second of the two electrodes. This allows detection of leakage when leakage forms a liquid path from the first to the second hole.

Such holes may particularly be large, e.g. larger than 500 nm, e.g. larger than 1 µm.

The recess structure may form one uniform cavity in the proximal surface, or it may form two or more separate cavities in the proximal surface. Herein, the first volume, *v1,* is the total volume of one or more separate cavities housing the two or more electrodes constituting the electrode structure.

The at least two electrodes may extend in the same cavity of the recess structure in the proximal surface. Particularly if the electrodes are intertwined wires, they extend in the same cavity. If the two electrodes are separated physically, i.e. not bonded to each other or intertwined, they may extend in separate cavities in the proximal surface.

Since the first volume, *v1,* exceeds the second volume, *v2,* the electrodes are at least partly surrounded by free space in the recess structure. The free space, which constitutes the third volume, *v3,* can be on one side of the electrodes, or different sides of the electrodes or on all sides of the electrodes, which would require the electrodes to be free-floating in the recess structure, e.g. suspended between a plurality of suspension points in the recess structure.

In one embodiment, the recess structure is completely, or partly, filled with a polymer, nonconductive material having superior water absorption properties compared with the water absorption properties of the base plate as such. This could be Polyvinylpyrrolidone (PVP) or similar well-known material.

The electrodes could be in contact with a bottom portion of the recess structure being furthest away from the proximal surface. The electrodes could be in continuous contact with the bottom portion throughout the length of the recess structure, or the electrodes could be discontinuously in contact with the bottom portion. In this case, the free space is mainly in the direction above the electrodes, i.e. between the electrodes and the proximal surface.

The recess structure may have a cross-sectional shape which could be square, rectangular, circular or any other shape. By cross-section, is herein meant a cross-section perpendicular to the axial direction of the recess structure and perpendicular to the proximal surface.

Particularly, the recess structure may have a Gaussian cross-sectional shape e.g. arising by composing an exponential function with a concave quadratic function.

The proximal surface may comprise an adhesive surface portion. The adhesive surface portion may particularly adhere the base plate to the skin surface of the user, and additionally, the adhesive surface portion may provide body compatibility, and it may further adhere the electrode structure in the recess structure.

The adhesive surface portion may be constituted by an adhesive layer made of a composition comprising one or more polyisobutenes and/or styrene-isoprene-styrene. The composition may comprise one or more hydrocolloids. The first composition may comprise one or more water soluble or water swellable hydrocolloids.

The composition may be a pressure sensitive adhesive composition suitable for medical purposes comprising a rubbery elastomeric base and one or more water soluble or water swellable hydrocolloids. The composition may comprise one or more polybutenes, one or more styrene copolymers, one or more hydrocolloids, or any combination thereof. The combination of the adhesive properties of the polybutenes and the absorbing properties of the hydrocolloids renders the composition suitable for use in ostomy appliances. The styrene copolymer may for example be a styrene-butadiene-styrene block copolymer or a styrene-isoprene-styrene block copolymer. Preferably, one or more styrene-isoprene-styrene (SIS) block type copolymers are employed. The amount of styrene block-copolymer may be from 5% to 20% of the total adhesive composition. The butene component is suitably a conjugated butadiene polymer selected from polybutadiene, polyisoprene. The polybutenes are preferably present in an amount of from 35 - 50% of the total adhesive composition. Preferably, the polybutene is polyisobutylene (PIB). Suitable hydrocolloids for incorporation in the composition are selected from naturally occurring hydrocolloids, semisynthetic hydrocolloids and synthetic hydrocolloids. The composition may comprise 20-60% hydrocolloids. A preferred hydrocolloid is carboxymethyl cellulose (CMC). The composition may optionally contain other components, such as fillers, tackifiers, plasticizers, and other additives.

Herein, the thickness direction is defined as a direction perpendicular to the proximal surface. The adhesive layer may have a substantially uniform thickness. The adhesive layer may have a thickness in the range from 0.1 mm to 1.5 mm, e.g. in the range from 0.2 mm to 1.2 mm, such as 0.8 mm or 1.0 mm.

Correspondingly, the recess structure may have a depth in the thickness direction being in the range of 0.5 mm to 1.5 mm, e.g. in the range from 0.7 mm to 1.2 mm, such as 0.8 mm or 1.0 mm.

The recess structure may particularly be provided in the adhesive surface portion such that the adhesive properties adheres the electrode structure in the recess structure. Accordingly, the electrode structure may be exposed towards the user in the direction towards the proximal surface. This may increase the signal strength and provide a faster electrical signal in response to humidity in the recess structure. In one embodiment, a first portion of the electrode structure is embedded in the composition of the adhesive layer, and a second portion of the electrode structure is not embedded in the composition of the adhesive layer. The second portion is between the first portion and the proximal surface.

The third volume, *v3,* may be at least equal to, or larger than the second volume, *v2*. In embodiments, the third volume, *v3,* is 1.2, 1.3, 1.4, 1.5, or 1.6 times the second volume, *v2,* or larger.

A releasable backing layer may cover at least a covered portion of the proximal surface. This may protect the base plate until intended use. In this embodiment, the backing layer may also cover the recess structure and electrode structure such that the electrode structure is not exposed towards the proximal surface until the backing layer is removed.

The base plate comprises an electronic control system configured for detection of humidity at the proximal surface based on an electrical signal from the electrode structure. The electronic control system may comprise a transmitter for transmitting electrical modulation signals to the electrode structure. Such a transmitter may be configured to apply a voltage between two of the at least two electrodes so as to establish an electrical circuit.

Additionally, the electronic control system comprises a receiver configured for determining resistance between the two electrodes. Particularly, the electronic control system should be configured to detect changes in a resistance between the electrodes and to activate an alarm when the changes of the resistance reach a predetermined value. In embodiments, the electronic control system is configured to detect a short-circuiting event across the two electrodes, the short-circuiting event being indicative of a liquid bridge formed in the interface between the base plate and the skin, and hence is indicative of the presence of output/leakage. The system can be further improved by defining two capacitors, one between one of the two electrodes and the surface of the body to which the base plate is attached, and a second capacitor between the other electrode and the surface of the body to which the base plate is attached. In that case, the electronic control system may be configured to determine variation between the two defined capacitances and to generate an alert when reaching a threshold value.

Irrespective whether one or two capacitor structures are defined, the resistance may change upon humidity in the recess structure and the recess structure may therefore improve the correctness of the activation of the alarm.

The proximal surface may extend in an interface plane and the electrode structure may extend at a distance from the interface plane. In one embodiment, the distance from the interface plane to all electrodes is the same. In another embodiment, one electrode is closer to the interface plane than other electrodes. In any event, the distance from the electrode or from all electrodes to the interface plane may be larger than the cross-sectional dimension, e.g. the diameter if the electrodes have circular cross-section. In that case, the distance between the skin surface of the person using the base plate and the closest electrode is larger than the cross-sectional dimension of the electrode and the risk of direct contact between skin surface and electrode is reduced.

In a second aspect, the disclosure provides an ostomy appliance comprising a base plate as described relative to the first aspect of the disclosure. The ostomy appliance further comprises a collection bag attached in communication with the stoma-receiving opening.

In a third aspect, the disclosure provides a method of manufacturing a base plate for detecting leakage, the method comprising:
- providing a base plate with a proximal surface configured to be attached to the body and defining a stoma-receiving opening from the proximal surface through the base plate; and
- providing an electrode structure comprising at least two electrodes,
- pressing the electrode structure into the proximal surface to thereby define a recess structure in the proximal surface for the electrode structure, the recess structure thereby defining a first volume, v1, which exceeds a second volume, v2, of the electrode structure, thereby defining a free space with a third volume, v3, around the electrode structure in the recess structure.

The step of pressing the electrode structure into the proximal surface could be carried out to cause elastic deformation of a first part of the base plate. That would inherently provide a shape with good flow properties, e.g. a Gaussian shape of the cross-section of the recess structure.

The step of pressing the electrode structure into the proximal surface may be carried out to cause plastic deformation of a second part of the base plate. That allows the recess structure to maintain its shape over time. The second part could be partly or completely the same as the first part of the base plate, or it could be two different parts of the base plate.

An adhesive, such as a pressure sensitive adhesive, could be applied to the proximal surface, and particularly to that part to which the electrode structure is applied such that the electrode structure is between the adhesive and the skin surface when the base plate is applied to the skin of a user.

Additionally, the disclosure may provide a method of using a base plate and an ostomy device as described herein. The method may include applying the base plate to the skin surface of a user, allowing humidity to generate in the recess structure, and generating an alert based on an electrical signal from the electrode structure.

### DETAILED DESCRIPTION

In the following, embodiments will be described in further details with reference to the drawing, in which:
Fig. 1 illustrates an embodiment of a base plate seen from the proximal surface,
Figs. 2-7 illustrate different embodiments of the base plate in a cross-section along section AA in Fig. 1; and
Fig. 8 illustrates a spiral shaped electrode and adhesive surface portions.

It should be understood that the detailed description and specific examples, while indicating embodiments, are given by way of illustration only, since various changes and modifications within the scope of the disclosure will become apparent to those skilled in the art from this detailed description.

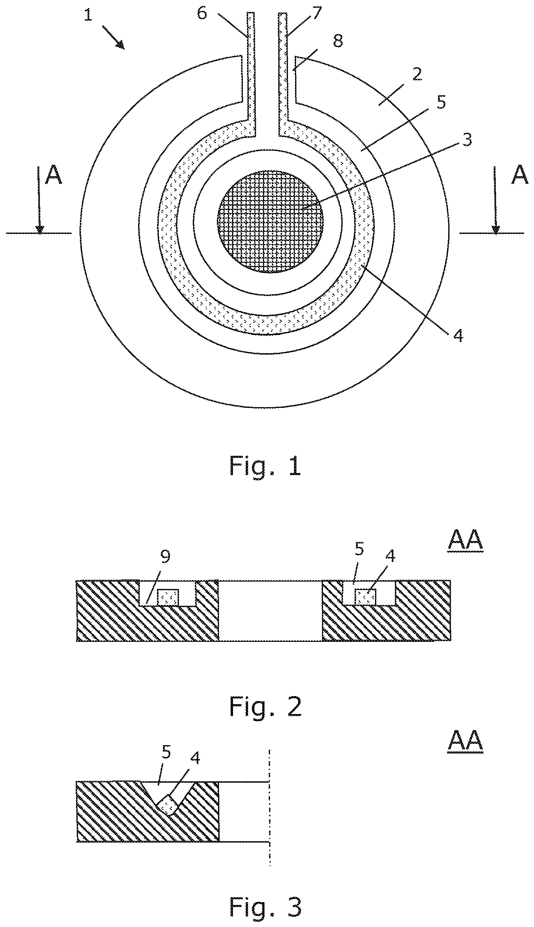

Fig. 1 illustrates a base plate 1 configured for use with or forming part of an ostomy appliance. The base plate has a proximal surface 2 provided with a layer of an adhesive material. The proximal surface is configured to be attached to the skin surface of the person using the ostomy appliance.

A stoma-receiving opening 3 extends from the proximal surface through the base plate and allows communication between the proximal surface and a stomal collection bag across the base plate.

The electrode structure 4 is, for simplicity, illustrated as one single element. It comprises at least two electrodes extending in the recess structure 5 in the proximal surface. The recess structure defines a first volume, *v1* which is larger than the second volume, *v2,* of the electrode and a free space is therefore defined in the recess structure. The free space is referred to herein as a third volume, *v3,* and it allows humidity to propagate around the electrode structure in the recess structure. The electrode structure 4 is connected to a control system via the connecting portions 6, 7 extending in a connecting recess structure 8 of the proximal surface 2.

The recess structure may have different shapes. Fig. 2 illustrates a cross-section along section AA in Fig. 1. In this embodiment, the recess structure has a rectangular cross-section. The electrode structure is, for simplicity, drawn as a rectangular element. Different shapes for the electrode structure may be considered, e.g. intertwined strands forming separate electrodes of the electrode structure. In the embodiment illustrated in Fig. 2, the free space is distributed on opposite sides and above the electrode structure, which is located against the bottom 9 of the recess structure

In Fig. 3 a half part from the edge to the centre line of the cross-section AA in Fig. 1 is illustrated for an embodiment, where the recess structure is parabolic. In this embodiment, a larger part of the free space is above the electrode structure and a smaller part is located on opposite sides of the recess structure.

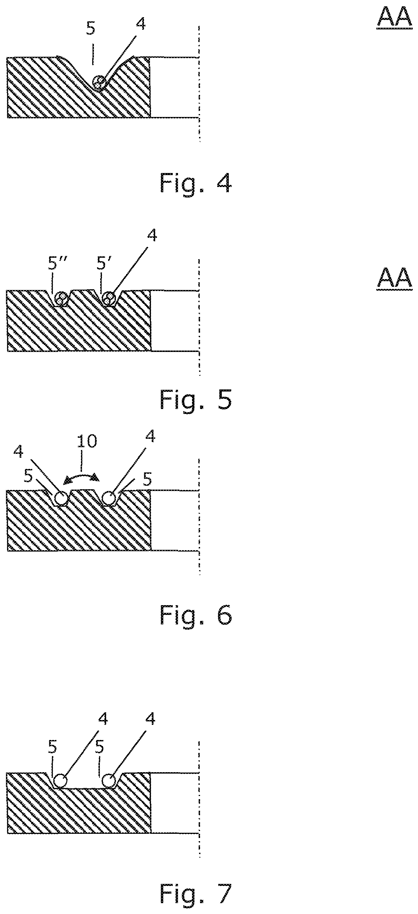

In Fig. 4 a half part from the edge to the centre line of the cross-section AA in Fig. 1 is illustrated for an embodiment, where the recess structure has a Gaussian cross-sectional shape. In this embodiment, a larger part of the free space is above the electrode structure and a smaller part is located on opposite sides of the recess structure like in Fig. 2 but the ability for liquid to flow along the proximal surface into the recess structure is improved due to the Gaussian curvature - particularly at the entrance into the recess structure. The electrode structure 4 in Fig. 4 comprises intertwined electrode strands, e.g. made of metal fibres. The electrode structure therefore constitutes a single intertwined electrode. The two intertwined electrodes are both coated and provided with holes in the coating, such that leakage/output can short-circuit the electrodes by establishing a liquid path from a hole in the coating of the first electrode to a hole in the coating of the second electrode.

In Fig. 5, the recess structures comprise two separate cavities 5' and 5" are provided, each comprising an electrode structure with two intertwined electrodes. In this embodiment, the electrode structure therefore constitutes two intertwined sets of electrodes. The two sets of intertwined electrodes are both coated and provided with holes in the coating, such that leakage/output can short-circuit the electrodes by establishing a liquid path from a hole in the coating of the first electrode to a hole in the coating of the second electrode, or upon short circuit from one cavity to the other cavity, i.e. between an electrode of one set of electrodes and an electrode of another set of electrodes.

In Fig. 6, two recess structures are provided, each comprising a single electrode, i.e. the electrode structure is spread into two recess structures. The electrical signal may be a result of a short circuiting between the recess structures as indicated by the arrow 10 due to presence of output (leakage) in the interface between the skin surface and the base plate. In Fig. 7, one recess structure is provided with an electrode structure forming two single electrodes spaced apart in the recess structure.

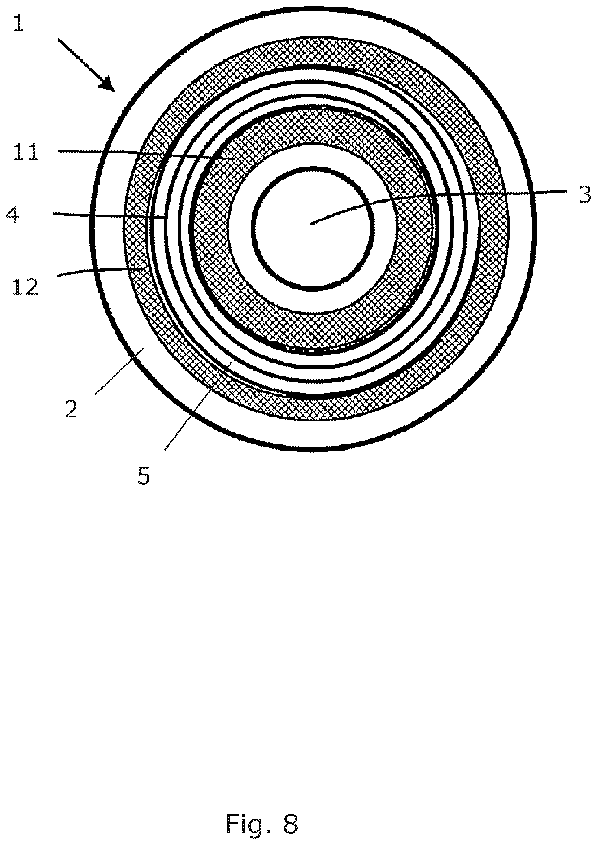

Fig. 8 illustrates an electrode structure 4 extending in the recess structure 5 in a spiral shape. The illustrated electrode structure could be made from intertwined wires etc. The illustrated base plate comprises two adhesive portions 11, 12 each constituted by a layer of an adhesive compound added to the proximal surface 2. In the illustrated embodiment, the adhesive portions are interrupted at the recess structure. In an alternative embodiment, the adhesive portion extends into the recess structure and may fully cover the recess structure such that the electrode structure can be fixed in the recess structure by adhesive properties of the adhesive layer.

An electronic control system configured for detection of humidity at the proximal surface based on an electrical signal from the electrode structure is described in detail inter alia in EP1991187.

Particularly, the system may be configured to provide an alert based on a signal from the electrode structure without direct physical or mechanical contact between the electrode structure and the skin surface of the user. This provides leak sensing without compromising the comfort of the user.

The electronic control system may be configured to apply a potential difference across the at least two electrodes. In particular, the electronic system may be configured to measure the resistance from one electrode to the other. In the event of a liquid path extending from one electrode to the other, a short-circuiting event can be detected due to a lowering of the resistance, e.g. approaching zero, and hence be indicative of the presence of output, i.e. a leakage.

## Claims

1. A base plate (1) for an ostomy appliance, the base plate forming a proximal surface (2) configured to be attached to the skin surface of a user and defining a stoma-receiving opening (3) from the proximal surface (2) through the base plate (1), wherein the base plate (1) comprises an electrode structure (4) comprising at least two electrodes extending in a recess structure (5) in the proximal surface (2), wherein the recess structure (5) defines a first volume, which exceeds a second volume of the electrode structure (4) to thereby define a free space with a third volume around the electrode structure (4) in the recess structure (5), **characterised in that** the recess structure (5) is in the proximal surface (2), the base plate further comprising an electronic control system configured for detection of humidity at the proximal surface (2) based on an electrical signal from the electrode structure (4), wherein the electronic control system comprises a receiver configured for determining resistance between the two electrodes.

2. The base plate according to claim 1, wherein the recess structure (5) has a Gaussian cross sectional shape.

3. The base plate (1) according to any of the preceding claims, wherein the proximal surface (2) comprises an adhesive surface portion.

4. The base plate (1) according to claim 3, wherein the recess structure (5) is provided in the adhesive surface portion.

5. The base plate (1) according to claim 4, wherein the electrode structure (4) is fixed in the recess structure (5) by adhesive properties of the adhesive surface portion.

6. The base plate (1) according to any of the preceding claims, wherein the third volume is equal to or larger than the second volume.

7. The base plate (1) according to any of the preceding claims, comprising: a backing layer covering at least a covered portion of the proximal surface (2), the recess structure (5) and electrode structure (4) being in the covered portion.

8. The base plate (1) according to any of the preceding claims, wherein the proximal surface (2) extends in an interface plane, and the electrode structure (4) extends at a distance from the interface plane.

9. The base plate (1) according to claim 8, wherein at least one of the electrodes has a cross-sectional dimension being smaller than the distance from the interface plane to the electrode.

10. An ostomy appliance comprising a base plate (1) according to any of claims 1-10 and a collection bag attached in communication with the stoma-receiving opening.

11. A method of manufacturing a base plate (1), the method comprising:
- providing a base plate (1) with a proximal surface (2) configured to be attached to the body and defining a stoma-receiving opening (3) from the proximal surface (2) through the base plate (1); and
- providing an electrode structure (4) comprising at least two electrodes,
- pressing the electrode structure (4) into the proximal surface (2) to thereby define a recess structure (5) in the proximal surface (2) for the electrode structure (4), the recess structure (5) thereby defining a first volume, which exceeds a second volume of the electrode structure, thereby defining a free space with a third volume around the electrode structure in the recess structure.

12. The method according to claim 11, wherein the step of pressing the electrode structure (4) into the proximal surface (2) is carried out to cause elastic deformation of a first part of the base plate (1).

13. The method according to claim 11 or 12, wherein the step of pressing the electrode structure (4) into the proximal surface (2) is carried out to cause plastic deformation of a second part of the base plate (1).

14. The method according to any of claims 11-13, comprising the step of applying a pressure sensitive adhesive to at least a part of the proximal surface (2) prior to the step of pressing the electrode structure (4) into that part of the proximal surface (2).

## Patentansprüche

1. Grundplatte (1) für eine Stomavorrichtung, wobei die Grundplatte eine proximale Oberfläche (2) bildet, die zum Befestigen an die Hautoberfläche eines Verwenders gestaltet ist und eine Stoma-aufnehmende Öffnung (3) von der proximalen Oberfläche (2) durch die Grundplatte (1) definiert, wobei die Grundplatte (1) eine Elektrodenstruktur (4) umfasst, die wenigstens zwei Elektroden umfasst, die in einer Vertiefungsstruktur (5) in der proximalen Oberfläche (2) verlaufen, wobei die Vertiefungsstruktur (5) ein erstes Volumen definiert, das ein zweites Volumen der Elektrodenstruktur (4) übersteigt, um dadurch einen freien Raum mit einem dritten Volumen um die Elektrodenstruktur (4) in der Vertiefungsstruktur (5) zu definieren, **dadurch gekennzeichnet, dass** die Vertiefungsstruktur (5) in der proximalen Oberfläche (2) angeordnet ist, wobei die Grundplatte ferner ein elektronisches Steuersystem umfasst, das zum Erfassen von Feuchtigkeit an der proximalen Oberfläche (2) auf der Grundlage eines elektrischen Signals von der Elektrodenstruktur (4) gestaltet ist, wobei das elektronische Steuersystem einen Empfänger umfasst, der zum Bestimmen des Widerstands zwischen den beiden Elektroden gestaltet ist.

2. Grundplatte gemäß Anspruch 1, wobei die Vertiefungsstruktur (5) eine Gauß'sche Querschnittsform aufweist.

3. Grundplatte (1) gemäß einem der vorstehenden Ansprüche, wobei die proximale Oberfläche (2) einen haftfähigen Oberflächenteil umfasst.

4. Grundplatte (1) gemäß Anspruch 3, wobei die Vertiefungsstruktur (5) in dem haftfähigen Oberflächenteil bereitgestellt ist.

5. Grundplatte (1) gemäß Anspruch 4, wobei die Elektrodenstruktur (4) in der Vertiefungsstruktur (5) durch Hafteigenschaften des haftfähigen Oberflächenteils fixiert ist.

6. Grundplatte (1) gemäß einem der vorstehenden Ansprüche, wobei das dritte Volumen gleich oder größer als das zweite Volumen ist.

7. Grundplatte (1) gemäß einem der vorstehenden Ansprüche, umfassend: eine Trägerschicht, die wenigstens einen bedeckten Teil der proximalen Oberfläche (2) bedeckt, wobei die Vertiefungsstruktur (5) und die Elektrodenstruktur (4) in dem bedeckten Teil liegen.

8. Grundplatte (1) gemäß einem der vorstehenden Ansprüche, wobei die proximale Oberfläche (2) in einer Grenzflächenebene verläuft und die Elektrodenstruktur (4) in einem Abstand von der Grenzflächenebene verläuft.

9. Grundplatte (1) gemäß Anspruch 8, wobei wenigstens eine der Elektroden eine Querschnittsabmessung aufweist, die kleiner als der Abstand von der Grenzflächenebene zu der Elektrode ist.

10. Stomavorrichtung, umfassend eine Grundplatte (1) gemäß einem der Ansprüche 1-10 und einen Sammelbeutel, der in Kommunikation mit der Stoma-aufnehmenden Öffnung befestigt ist.

11. Verfahren zur Herstellung einer Grundplatte (1), wobei das Verfahren umfasst:
- Bereitstellen einer Grundplatte (1) mit einer proximalen Oberfläche (2), die zum Befestigen an den Körper gestaltet ist und eine Stoma-aufnehmende Öffnung (3) von der proximalen Oberfläche (2) durch die Grundplatte (1) definiert, und
- Bereitstellen einer Elektrodenstruktur (4), die wenigstens zwei Elektroden umfasst,
- Pressen der Elektrodenstruktur (4) in die proximale Oberfläche (2), um dadurch eine Vertiefungsstruktur (5) in der proximalen Oberfläche (2) für die Elektrodenstruktur (4) zu definieren, wobei die Vertiefungsstruktur (5) dadurch ein erstes Volumen definiert, das ein zweites Volumen der Elektrodenstruktur übersteigt, um dadurch einen freien Raum mit einem dritten Volumen um die Elektrodenstruktur in der Vertiefungsstruktur zu definieren.

12. Verfahren gemäß Anspruch 11, wobei der Schritt des Pressens der Elektrodenstruktur (4) in die proximale Oberfläche (2) durchgeführt wird, um elastische Verformung eines ersten Teils der Grundplatte (1) zu bewirken.

13. Verfahren gemäß Anspruch 11 oder 12, wobei der Schritt des Pressens der Elektrodenstruktur (4) in die proximale Oberfläche (2) durchgeführt wird, um plastische Verformung eines zweiten Teils der Grundplatte (1) zu bewirken.

14. Verfahren gemäß einem der Ansprüche 11-13, umfassend den Schritt des Aufbringens eines Haftklebers auf wenigstens einen Teil der proximalen Oberfläche (2) vor dem Schritt des Pressens der Elektrodenstruktur (4) in diesen Teil der proximalen Oberfläche (2).

## Revendications

1. Plaque de base (1) pour un appareillage de stomie, la plaque de base formant une surface proximale (2) configurée pour être attachée à la surface de la peau d'un utilisateur et définissant une ouverture de réception de stomie (3) à partir de la surface proximale (2) à travers la plaque de base (1), la plaque de base (1) comprenant une structure d'électrode (4) comprenant au moins deux électrodes s'étendant dans une structure d'évidement (5) dans la surface proximale (2), la structure d'évidement (5) définissant un premier volume, qui dépasse un deuxième volume de la structure d'électrode (4) pour définir ainsi un espace libre avec un troisième volume autour de la structure d'électrode (4) dans la structure d'évidement (5), **caractérisée en ce que** la structure d'évidement (5) se trouve dans la surface proximale (2), la plaque de base comprenant en outre un système de commande électronique configuré pour la détection de l'humidité au niveau de la surface proximale (2) sur la base d'un signal électrique provenant de la structure d'électrode (4), le système de commande électronique comprenant un récepteur configuré pour déterminer la résistance entre les deux électrodes.

2. Plaque de base selon la revendication 1, la structure d'évidement (5) ayant une forme de section transversale gaussienne.

3. Plaque de base (1) selon l'une quelconque des revendications précédentes, la surface proximale (2) comprenant une partie de surface adhésive.

4. Plaque de base (1) selon la revendication 3, la structure d'évidement (5) étant prévue dans la partie de surface adhésive.

5. Plaque de base (1) selon la revendication 4, la structure d'électrode (4) étant fixée dans la structure d'évidement (5) par des propriétés adhésives de la partie de surface adhésive.

6. Plaque de base (1) selon l'une quelconque des revendications précédentes, le troisième volume étant égal ou supérieur au deuxième volume.

7. Plaque de base (1) selon l'une quelconque des revendications précédentes, comprenant : une couche de support recouvrant au moins une partie couverte de la surface proximale (2), la structure d'évidement (5) et la structure d'électrode (4) se trouvant dans la partie couverte.

8. Plaque de base (1) selon l'une quelconque des revendications précédentes, la surface proximale (2) s'étendant dans un plan d'interface, et la structure d'électrode (4) s'étendant à une certaine distance du plan d'interface.

9. Plaque de base (1) selon la revendication 8, au moins une des électrodes ayant une dimension de section transversale qui est plus petite que la distance entre le plan d'interface et l'électrode.

10. Appareillage de stomie comprenant une plaque de base (1) selon l'une quelconque des revendications 1 à 10 et un sac de collecte attaché en communication avec l'ouverture réceptrice de stomie.

11. Procédé de fabrication d'une plaque de base (1), le procédé comprenant :
- la fourniture d'une plaque de base (1) avec une surface proximale (2) configurée pour être attachée au corps et définir une ouverture réceptrice de stomie (3) à partir de la surface proximale (2) à travers la plaque de base (1) ; et
- la fourniture d'une structure d'électrode (4) comprenant au moins deux électrodes,
- le pressage de la structure d'électrode (4) dans la surface proximale (2) pour définir ainsi une structure d'évidement (5) dans la surface proximale (2) pour la structure d'électrode (4), la structure d'évidement (5) définissant ainsi un premier volume, qui dépasse un deuxième volume de la structure d'électrode, définissant ainsi un espace libre avec un troisième volume autour de la structure d'électrode dans la structure d'évidement.

12. Procédé selon la revendication 11, l'étape de pressage de la structure d'électrode (4) dans la surface proximale (2) étant effectuée pour provoquer une déformation élastique d'une première partie de la plaque de base (1).

13. Procédé selon la revendication 11 ou 12, l'étape de pressage de la structure d'électrode (4) dans la surface proximale (2) étant effectuée pour provoquer une déformation plastique d'une deuxième partie de la plaque de base (1).

14. Procédé selon l'une des revendications 11 à 13, comprenant l'étape d'application d'un adhésif sensible à la pression sur au moins une partie de la surface proximale (2) avant l'étape de pressage de la structure d'électrode (4) dans cette partie de la surface proximale (2).
